Europäisches Patentamt

**European Patent Office**

**Office européen des brevets**

(19)

(11) Publication number: **0 022 576**
A1

(12) **EUROPEAN PATENT APPLICATION**

(21) Application number: **80104025.4**

(22) Date of filing: **11.07.80**

(51) Int. Cl.³: **A 61 M 5/34**

(30) Priority: **11.07.79 DE 7919910 U**

(43) Date of publication of application: **21.01.81**
**Bulletin 81/3**

(84) Designated Contracting States: **AT BE CH DE FR GB IT LI LU NL SE**

(71) Applicant: **von Heyden GmbH, Volkartstrasse 83, D-8000 Munich 19 (DE)**

(72) Inventor: **Geismar, Wilhelm, Dr., Kueffnerstrasse 2, D-8400 Regensburg (DE)**
Inventor: **Rapp, Hans, Destouchesstrasse 59, D-8000 Munich 40 (DE)**
Inventor: **Ferme, Vincenzo, Erlenstrasse 3, D-8411 Waldetzenberg (DE)**

(74) Representative: **Vossius-Vossius-Tauchner-Heunemann-Rauh Patentanwälte, P.O.Box 860767 Siebertstrasse 4, D-8000 München 86 (DE)**

(54) **Syringe with inner cone.**

(57) In a syringe comprising a syringe body (1) a sealable cone member (4) is disposed at the connector end (2) of said syringe body (1), said cone member (4) exhibiting an axial bore (5) and acting as an adapter onto which a needle can be affixed. In order to reduce the number of individual parts and so simplify manufacture and assembly, the cone member (4) is inserted through the bore of the connector end (2) and at its base exhibits a flange (6) which makes it sit tightly against the inner wall of the syringe body (1). The cone member (4) is preferably inserted from the inside of the syringe body (1) out through the bore of the connector end (2).

EP 0 022 576 A1

Our Ref.: P 731 EP

SYRINGE WITH INNER CONE

This invention relates to a syringe comprising a syringe body and a sealable cone member with an axial bore as adapter onto which a needle can be affixed. Syringes of this type are used in medicine especially for injecting liquids or gases and for puncture.

Usually such syringes consist of a graduated clear glass cylinder having a terminal, centrally or eccentrically disposed, standardized positive cone as needle neck, e.g. a Luer-Lock neck or record neck, together with a piston section. This needle neck forms the adapter between the syringe body and the injecting needle to be affixed onto the body. In conventional syringes this adapter is made up of several parts, namely of a cone member incorporating a flange, which is pushed onto the tapered connector end of the syringe body with a ring gasket in between, and of a sleeve by means of

0022576

which the cone member is locked and sealed at the connector end of the syringe body. Because so many individual parts are required, such syringes are expensive to manufacture, both with regard to the manufacture of the individual parts and the assembly of the syringe. This proves to be a particular drawback in the case of the disposable syringes which are in common use.

By contrast, the object of the present invention is to provide a relatively inexpensive syringe, the prime consideration in accomplishing this object being to reduce the number of individual parts required for the adapter.

The solution provided by the present invention is distinguished by the fact that at its base the cone member exhibits a flange to ensure that it sits snugly against the inner wall of the syringe body. The adapter of the invention is made up of a single part, thus simplifying both the manufacture and the assembly of the syringe. Thanks to the fact that the flange fits against the inner wall of the syringe body, i.e. the cone member is pushed from the interior of the syringe body out through the connector end until the flange rests against the inner wall of the syringe body, on the one hand an effective seal is achieved between the cone member and the syringe body, and on the other hand the flange at the same time forms an abutment so that the cone member cannot be pressed or drawn outward out of the connector end of the syringe body. Rather, thanks to a corresponding action of pressure or suction, the snug location of the flange against the inner wall of the syringe body - and hence the seal - is further improved.

In the syringe of this invention an annular collar can additionally be provided on the outer circumference of the cone member, so that after being pushed through the connector end of the syringe body the cone member is locked tight between the collar and the flange.

0022576

Furthermore, an additional sealing collar may advantageously
be provided on the outer circumference of the cone member
between the collar and the flange, said sealing collar improving the seal between the connector end of the syringe body
and the cone member.

As in the conventional cone members, a sealing cap may advantageously also be pushed onto the cone member of the syringe
of this invention, so that the syringe is kept sealed during
storage and the cone member protected.

The sealing cap and the cone member are advantageously dimensioned such that the two components can be affixed together
before being introduced into the syringe, and so assembled
can be pushed together from inside the syringe out into the
bore of the connector end.  In this way assembly is simplified.
The inner surface of the sealing cap can be of a conical design, this surface being complementary to the outer surface of
the cone member, so that a very tight seal can be achieved
when locked.  This sealing action can be further improved by
providing the sealing cap with an axial sealing pin which can
be introduced into the axial bore of the cone member, the point
where this pin connects with the sealing cap preferably being
complementarily conical to the inner cone at the free end of
the cone member.  In order to prevent the sealing cap from
undesirably becoming detached, an interlocking arrestor connection  can be provided on this sealing cap and on the cone
member, said arrestor connection preferably exhibiting a collar
ring on the cone member and a corresponding annular groove on
the sealing cap, or vice versa.

In accordance with the present invention the bore of the cone
member can further be sealed by means of a diaphragm, for
instance in the region of the flange, for example to prevent
the inside of the syringe from becoming contaminated during storage.  Before the syringe is used this diaphragm is then torn or punctured, for example by the pressure released when the syringe piston is actuated, or else by pressing onto it with the sealing cap pin or some other needle-shaped object.

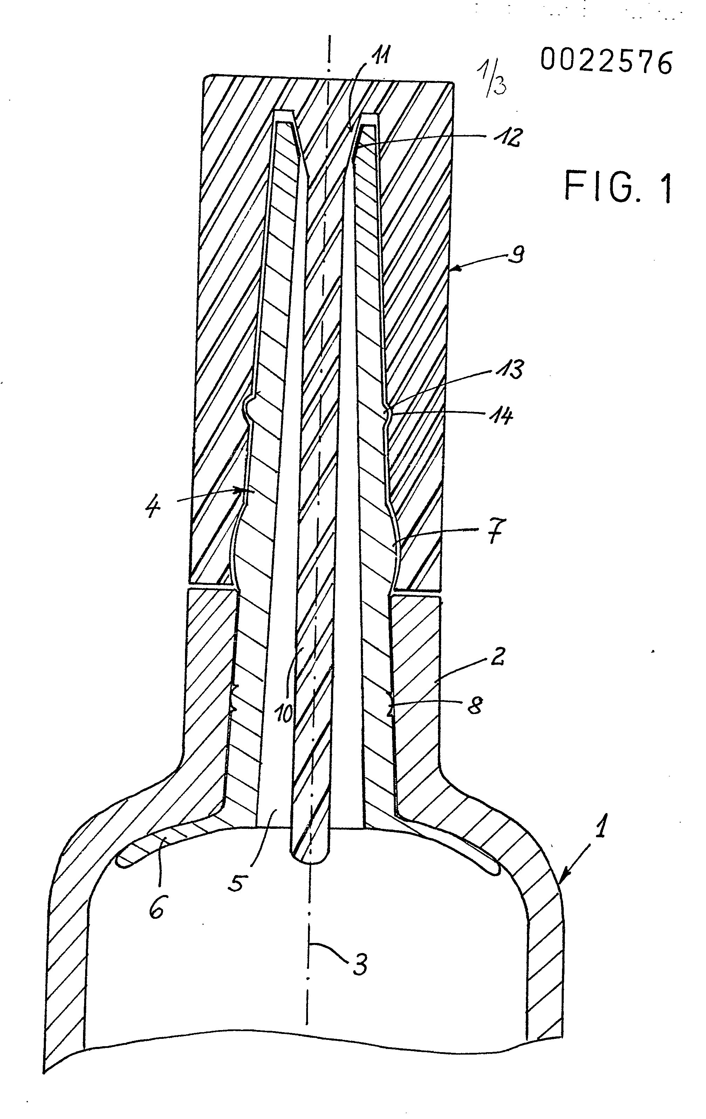

The invention is explained in greater detail below with reference to the attached drawing.  Figs. 1 to 3  each show a longitudinal section of part of two embodiments of the syringe of this invention with a syringe body 1, cone member 4 and sealing cap 9.

The essentially cylindrical syringe body 1 exhibits a tapered connector end 2 for a needle or for the cap.  Like the syringe body 1, this connector end 2 is also of an essentially cylindrical or conical design and in the embodiment illustrated is coaxial to the syringe body.  The common axis is designated by reference numeral 3.  Alternatively, however, the connector end 2, for example, can also be disposed eccentrically to the syringe body 1.  The cone member 4 with its axial bore 5 exhibits a flange 6 at its base, which is so designed that it sits tightly against the inner wall of the syringe body 1 when introduced into the cone member 4 via the connector end 2 of the syringe body 1.  Thus it is advantageous if the cone member and especially the flange 6 are manufactured from plastically and/or elastically ductile material so as to ensure that the flange sits tightly against the syringe body.

In the embodiment shown in Fig. 1 an annular collar 7 is formed on the outer circumference of the cone member 4 to permit the cone member 4 to lock between the collar 7 and the flange 6.  To this end, the distance between the collar 7 and the flange 6 is selected according to the length of the connector end 2.

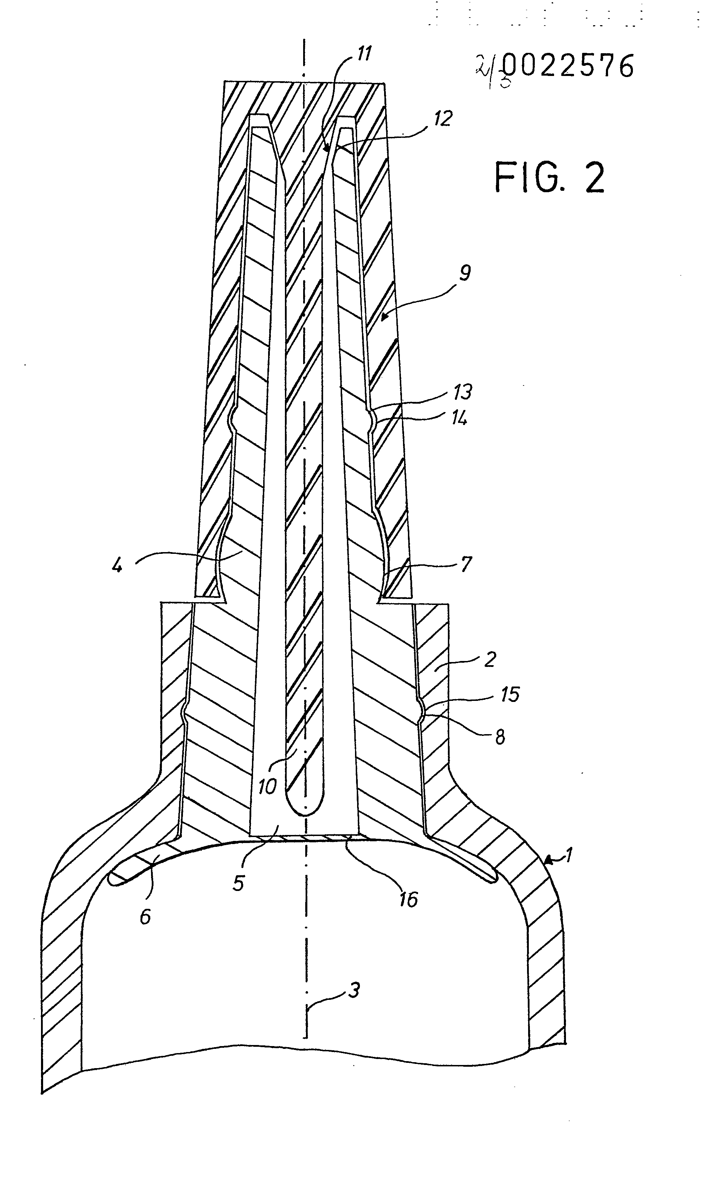

The outer surface of the cone member 4 between the collar 7 and the flange 6 can be of a conical or cylindrical design, though a slight conical tapering toward the collar 7 is preferable in order to make it easier to introduce the cone member 4 into the connector end 2. To increase the sealing action between the cone member 4 and the syringe body 1, an additional
annular sealing collar 8 can be disposed on the outer circumference of the cone member 4 between the collar 7 and
the flange 6, which sealing collar, as shown in Fig. 2,
advantageously engages in a corresponding inner annular
groove 15 in the connector end 2 so as to improve location
of the cone member 4 in the syringe body.

The free end of the cone member 4 adjoining
the collar 7 is designed to be essentially complementary to
the neck surface of the needle to be affixed, for example corresponding to the Luer-Lock neck or record neck already mentioned.
The sealing cap 9, which is designed to seal off the syringe
before use, is correspondingly shaped. This sealing cap 9
exhibits a central sealing pin 10 which extends down coaxially
into the axial bore of the cone member 4 when the sealing cap
9 is affixed. Since the cone member 4 exhibits an inner cone
12 at its free end, the neck 11 of the sealing pin 10 on the
sealing cap 9 is designed to be complementarily conical to
the inner cone 12 so that the sealing action is increased when
the sealing cap 9 is affixed. In order to prevent the sealing
cap 9 from being unintentionally pulled off  the cone member
4, an arrestor connection is provided between the two, this
arrestor connection for example being formed by a collar ring
13 on the outer circumference of the cone member 4 and by a
corresponding annular groove 14 in the sealing cap 9. The
shape of the axial bore 5, for example cylindrical or conical,
can be selected according to the desired conditions; in the
case of the embodiment depicted the axial bore 5 is conical
and tapers toward the free end of the cone member 4.

The cone member 4 of the invention, which serves as an adap-
ter  between the syringe body 1 and the needle (not illustrated), is designed as a single part, with the result that both
the manufacture and the assembly of the syringe are simplified.

The cone member 4 and/or the sealing cap 9 can be manufactured quite easily from plastic, e.g. a hard rubber such
as polyethylene, for instance by injection molding.

In the embodiment as shown in Fig. 1 the syringe is assembled by first of all pushing the cone member 4 from inside
into the bore of the connector end 2 of the syringe body 1,
and then the sealing cap 9 is affixed from outside. Conversely, in the embodiment shown in Fig. 2 the cone member 4
and the sealing cap 9 can first be assembled and then together
pushed from inside the syringe body 1 out into the bore of
the connector end 2.

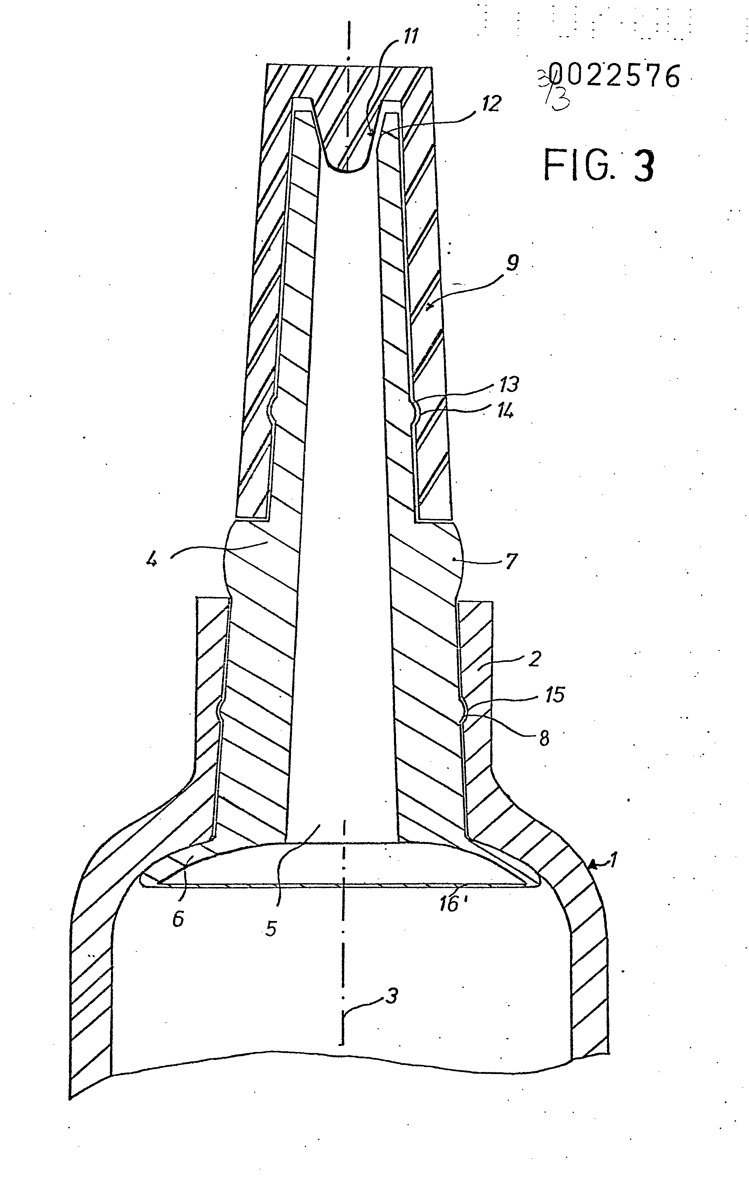

Furthermore, according to Figs. 2 and 3 a diaphragm 16, 16'
for sealing off the axial bore 5 of the cone member 4 can be
designed in one piece with the latter; this diaphragm ensures
that the syringe body is completely sealed hermetically until
used. Only when the syringe is used is the diaphragm torn
due to the application of pressure by actuating the syringe
piston (not illustrated) or else punctured using a needle
or the sealing pin 10 of the sealing cap 9. The diaphragm
can be made from the same material as the cone member 4 or
from different material, for example in the form of a one-
piece plastic injection molded part. If the diaphragm is
disposed in the region of the flange 6, the sealing pin 10
of the sealing cap 9, or even the latter itself, can be dispensed with (e.g. as in Fig. 3), since thanks to the diaphragm
16, 16' injection substances from the syringe body 1 are prevented from penetrating into the axial bore 5 of the cone
member 4, which could otherwise lead to blockages there.

In the embodiment according to Fig. 3, unlike in Fig. 2,
the sealing cap 9 is shortened at its lower end and the collar 7
enlarged such that, as in the embodiment of Fig. 1, it helps to
seal the cone member 4     at the connector end 2. It is therefore possible in this case to dispense with the arrestor
connection 8, 15, if so desired.

0022576

WHAT IS CLAIMED IS:

1.  Syringe comprising a syringe body (1) and a sealable cone member (4) disposed at the connector end (2) of said syringe body (1), said cone member (4) exhibiting an axial bore (5) and acting as an adapter onto which a needle can be affixed, wherein the cone member (4) is inserted through the bore of the connector end (2) and at its base exhibits a flange (6) which makes it sit tightly against the inner wall of the syringe body (1).

2.  Syringe as in claim 1 wherein the cone member (4) is inserted from the inside of the syringe body (1) through the bore of the connector end (2).

3.  Syringe as in claims 1 or 2 wherein the cone member (4) exhibits an annular collar (7) on its outer circumference for locking the cone member (4) between the inner wall of the syringe body (1) and the free connector end (2).

4.  Syringe as in any of claims 1 to 3 wherein between the collar (7) and the flange (6) a sealing collar (8) is disposed on the outer circumference of the cone member (4).

5.  Syringe as in any of claims 1 to 4 wherein a sealing cap (9) can be pushed onto the cone member (4) to seal the latter.

6.  Syringe as in claim 5 wherein the cone member (4) together with the affixed sealing cap (9) can be pushed out into the bore of the connector end (2).

7.  Syringe as in claims 5 or 6 wherein the inner surface of the sealing cap (9) is of a conical design corresponding to the outer surface of the cone member (4).

8.  Syringe as in any of claims 5 to 7 wherein the sealing cap

- 8 -

0022576

(9) exhibits an axial sealing pin (10) which can be introduced into the axial bore (5) of the cone member (4).

9.   Syringe as in claim 8 wherein the neck (11) of the sealing pin (10) on the sealing cap (9) is of a conical design
corresponding to the inner cone (12) of the cone member (4).

10.   Syringe as in any of claims 5 to 9 wherein the cone
member (4) and the sealing cap (9) exhibit an interlocking
arrestor connection (13,14).

11.   Syringe as in claim 10 wherein the arrestor connection
exhibits a collar ring (13) on the sealing cap (9) and a
corresponding annular groove (14) on the cone member (4),
or vice versa.

12.   Syringe as in any of claims 1 to 11 wherein the axial
bore (5) of the cone member (4) is sealed by a diaphragm
(16,16') which can be torn or punctured before the syringe
is used.

13.   Syringe as in claim 12 wherein the diaphragm (16,16')
is disposed in the region of the flange (6).

14.   Syringe as in claim 13 wherein the diaphragm (16') is
joined to the outer  edge   of the flange (6).

15.   Syringe as in any of claims 4 to 14 wherein the collar
(8) of the cone member (4) forms an arrestor connection together with an inner annular groove (15) of the connector end
(2) of the syringe body (1).

0022576

FIG. 1

FIG. 2

FIG. 3

**European Patent Office**

**EUROPEAN SEARCH REPORT**

0022576

Application number

EP 80 10 4025

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| X | FR - A - 1 035 398 (MEDIGLASS) <br> * Page 2, column 1, paragraphs 2 and 3 * <br> -- | 1-3 | A 61 M 5/34 |
| X | US - A - 1 705 525 (HOFSCHNEIDER) <br> * Page 2, lines 76-97; figures 1-3 * <br> -- | 1,2,4 | |
| | US - A - 3 234 944 (STEVENS) <br> * Column 5, lines 1-44; figure 4 * <br> -- | 1,2 | TECHNICAL FIELDS SEARCHED (Int. Cl.³) |
| | US - A - 1 508 936 (PLATT) <br> * Claims; figures * <br> -- | 1,2,9 | A 61 M |
| | US - A - 3 523 531 (BURKE) <br> * Claims; figures * <br> -- | 1,2,4, 15 | |
| | FR - A - 2 186 270 (PHILIPS) <br> * Page 3, lines 31-38; page 4, line 1 * <br> ---- | 5,7,8, 12 | CATEGORY OF CITED DOCUMENTS <br> X: particularly relevant <br> A: technological background <br> O: non-written disclosure <br> P: intermediate document <br> T: theory or principle underlying the invention <br> E: conflicting application <br> D: document cited in the application <br> L: citation for other reasons <br> &: member of the same patent family, corresponding document |

The present search report has been drawn up for all claims

| Place of search | Date of completion of the search | Examiner |
|---|---|---|
| The Hague | 29-09-1980 | VANRUNXT |

EPO Form 1503.1  06.78